Europäisches Patentamt

European Patent Office

Office européen des brevets

(11) Publication number: 0 549 675 B1

# EUROPEAN PATENT SPECIFICATION

(45) Date of publication of patent specification: 05.04.95

(51) Int. Cl.⁶: **C11D 3/386**, C07K 14/81, C12N 9/96, C12N 15/00

(21) Application number: 91916862.5

(22) Date of filing: 18.09.91

(86) International application number:
PCT/DK91/00279

(87) International publication number:
WO 92/05239 (02.04.92 92/08)

(54) **DETERGENT CONTAINING PROTEASE AND INHIBITOR AND NOVEL INHIBITORS FOR USE THEREIN.**

(30) Priority: **18.09.90 DK 2237/90**

(43) Date of publication of application:
**07.07.93 Bulletin 93/27**

(45) Publication of the grant of the patent:
**05.04.95 Bulletin 95/14**

(84) Designated Contracting States:
**AT BE CH DE DK ES FR GB GR IT LI LU NL SE**

(56) References cited:
**WO-A-92/03529**
**US-A- 5 039 446**

**PATENT ABSTRACTS OF JAPAN, Vol 12, No 155, C494, Abstract of JP 62269689, publ 1987-11-24 (SHOWA DENKO K.K.).**

(73) Proprietor: **NOVO NORDISK A/S**
**Novo Allé**
**DK-2880 Bagsvaerd (DK)**

(72) Inventor: **MIKKELSEN, Jan, Moeller**
**Snerlevej 20**
**DK-2820 Gentofte (DK)**
Inventor: **SVENDSEN, Allan**
**Bakkeleddet 28**
**DK-3460 Birker d (DK)**
Inventor: **DIDERICHSEN, Boerge**
**Fuglesangsvej 4**
**DK-3460 Birkeroed (DK)**
Inventor: **CLAUSEN, Ib, Groth**
**Fyrrestien 6**
**DK-3400 Hilleroed (DK)**

(74) Representative: **Thalsoe-Madsen, Kine Birgit et al**
**c/o Novo Nordisk A/S**
**Novo Allé**
**DK-2880 Bagsvaerd (DK)**

SEQUENCE LISTING

(1) GENERAL INFORMATION:

    (i) APPLICANT: Novo Nordisk A/S

    (ii) TITLE OF INVENTION: Detergent containing protease and
          inhibitor and novel inhibitor

   (iii) NUMBER OF SEQUENCES: 4

    (iv) CORRESPONDENCE ADDRESS:
        (A) ADDRESSEE: Novo Nordisk A/S
        (B) STREET: Novo Alle
        (C) CITY: Bagsvaerd
        (E) COUNTRY: Denmark
        (F) ZIP: 2880

    (v) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25

    (vi) CURRENT APPLICATION DATA:
        (A) APPLICATION NUMBER:
        (B) FILING DATE:
        (C) CLASSIFICATION:

  (viii) ATTORNEY/AGENT INFORMATION:
        (A) NAME: Thalsoe-Madsen, Birgit
        (C) REFERENCE/DOCKET NUMBER: 3486.204-WO

    (ix) TELECOMMUNICATION INFORMATION:
        (A) TELEPHONE: +4544448888
        (B) TELEFAX: +4544493256
        (C) TELEX: 37304

(2) INFORMATION FOR SEQ ID NO:1:

    (i) SEQUENCE CHARACTERISTICS:
        (A) LENGTH: 592 base pairs
        (B) TYPE: nucleic acid
        (C) STRANDEDNESS: single
        (D) TOPOLOGY: linear

    (ii) MOLECULE TYPE: cDNA

    (vi) ORIGINAL SOURCE:
        (A) ORGANISM: barley

    (ix) FEATURE:
        (A) NAME/KEY: CDS
        (B) LOCATION: 77..580

```
        (ix)  FEATURE:
              (A)  NAME/KEY: sig_peptide
              (B)  LOCATION: 77..331

        (ix)  FEATURE:
              (A)  NAME/KEY: mat_peptide
              (B)  LOCATION: 332..580


        (xi) SEQUENCE DESCRIPTION: SEQ ID NO:1:

GAATTCCATT CAAGAATAGT TCAAACAAGA AGATTACAAA CTATCAATTT CATACACAAT        60

ATAAACGATT AAAAGA ATG AGA TTT CCT TCA ATT TTT ACT GCA GTT TTA          109
                  Met Arg Phe Pro Ser Ile Phe Thr Ala Val Leu
                  -85              -80                 -75

TTC GCA GCA TCC TCC GCA TTA GCT GCT CCA GTC AAC ACT ACA ACA GAA        157
Phe Ala Ala Ser Ser Ala Leu Ala Ala Pro Val Asn Thr Thr Thr Glu
             -70              -65              -60

GAT GAA ACG GCA CAA ATT CCG GCT GAA GCT GTC ATC GGT TAC TCA GAT        205
Asp Glu Thr Ala Gln Ile Pro Ala Glu Ala Val Ile Gly Tyr Ser Asp
             -55              -50              -45

TTA GAA GGG GAT TTC GAT GTT GCT GTT TTG CCA TTT TCC AAC AGC ACA        253
Leu Glu Gly Asp Phe Asp Val Ala Val Leu Pro Phe Ser Asn Ser Thr
             -40              -35              -30

AAT AAC GGG TTA TTG TTT ATA AAT ACT ACT ATT GCC AGC ATT GCT GCT        301
Asn Asn Gly Leu Leu Phe Ile Asn Thr Thr Ile Ala Ser Ile Ala Ala
         -25              -20              -15

AAA GAA GAA GGG GTA TCT TTG GAT AAA AGA AGT TCA GTG GAG AAG AAG        349
Lys Glu Glu Gly Val Ser Leu Asp Lys Arg Ser Ser Val Glu Lys Lys
-10               -5                  1                   5

CCC GAG GGA GTG AAC ACC GGT GCT GGT GAC CGT CAC AAC CTG AAG ACA        397
Pro Glu Gly Val Asn Thr Gly Ala Gly Asp Arg His Asn Leu Lys Thr
             10               15               20

GAG TGG CCA GAG TTG GTG GGG AAA TCG GTG GAG GAG GCC AAG AAG GTG        445
Glu Trp Pro Glu Leu Val Gly Lys Ser Val Glu Glu Ala Lys Lys Val
         25               30               35

ATT CTG CAG GAC AAG CCA GAG GCG CAA ATC ATA GTT CTG CCG GTG GGT        493
Ile Leu Gln Asp Lys Pro Glu Ala Gln Ile Ile Val Leu Pro Val Gly
     40               45               50

ACC ATT GTG ACC ATG GAA TAT CGG ATC GAT CGC GTC CGC CTC TTT GTC        541
Thr Ile Val Thr Met Glu Tyr Arg Ile Asp Arg Val Arg Leu Phe Val
 55               60               65               70
```

GAT AAA CTC GAC AAC ATT GCC CAG GTC CCT AGG GTC GGC TAGTGATCTA          590
Asp Lys Leu Asp Asn Ile Ala Gln Val Pro Arg Val Gly
                    75                  80

GA                                                                     592


(2) INFORMATION FOR SEQ ID NO:2:

        (i) SEQUENCE CHARACTERISTICS:
            (A) LENGTH: 168 amino acids
            (B) TYPE: amino acid
            (D) TOPOLOGY: linear

        (ii) MOLECULE TYPE: protein

        (xi) SEQUENCE DESCRIPTION: SEQ ID NO:2:

Met Arg Phe Pro Ser Ile Phe Thr Ala Val Leu Phe Ala Ala Ser Ser
-85                 -80             -75             -70

Ala Leu Ala Ala Pro Val Asn Thr Thr Thr Glu Asp Glu Thr Ala Gln
            -65             -60             -55

Ile Pro Ala Glu Ala Val Ile Gly Tyr Ser Asp Leu Glu Gly Asp Phe
        -50             -45             -40

Asp Val Ala Val Leu Pro Phe Ser Asn Ser Thr Asn Asn Gly Leu Leu
        -35             -30             -25

Phe Ile Asn Thr Thr Ile Ala Ser Ile Ala Ala Lys Glu Glu Gly Val
        -20             -15             -10

Ser Leu Asp Lys Arg Ser Ser Val Glu Lys Lys Pro Glu Gly Val Asn
 -5                 1               5                   10

Thr Gly Ala Gly Asp Arg His Asn Leu Lys Thr Glu Trp Pro Glu Leu
            15              20              25

Val Gly Lys Ser Val Glu Glu Ala Lys Lys Val Ile Leu Gln Asp Lys
        30              35              40

Pro Glu Ala Gln Ile Ile Val Leu Pro Val Gly Thr Ile Val Thr Met
    45              50              55

Glu Tyr Arg Ile Asp Arg Val Arg Leu Phe Val Asp Lys Leu Asp Asn
 60              65              70              75

Ile Ala Gln Val Pro Arg Val Gly
                80

(2) INFORMATION FOR SEQ ID NO:3:

        (i) SEQUENCE CHARACTERISTICS:
            (A) LENGTH: 336 base pairs
            (B) TYPE: nucleic acid
            (C) STRANDEDNESS: single

(D) TOPOLOGY: linear

(ii) MOLECULE TYPE: cDNA

(vi) ORIGINAL SOURCE:
    (A) ORGANISM: barley

(ix) FEATURE:
    (A) NAME/KEY: CDS
    (B) LOCATION: 10..324

(ix) FEATURE:
    (A) NAME/KEY: sig_peptide
    (B) LOCATION: 10..75

(ix) FEATURE:
    (A) NAME/KEY: mat_peptide
    (B) LOCATION: 76..324

(xi) SEQUENCE DESCRIPTION: SEQ ID NO:3:

```
GGATCCACC ATG AGG AGC TCC CTT GTG CTG TTC TTT GTC TCT GCG TGG          48
          Met Arg Ser Ser Leu Val Leu Phe Phe Val Ser Ala Trp
          -22     -20                 -15             -10

ACG GCC TTG GCC AGT CCT ATT CGT CGA AGC TCA GTG GAG AAG AAG CCC       96
Thr Ala Leu Ala Ser Pro Ile Arg Arg Ser Ser Val Glu Lys Lys Pro
                -5                  1               5

GAG GGA GTG AAC ACC GGT GCT GGT GAC CGT CAC AAC CTG AAG ACA GAG      144
Glu Gly Val Asn Thr Gly Ala Gly Asp Arg His Asn Leu Lys Thr Glu
        10                  15              20

TGG CCA GAG TTG GTG GGG AAA TCG GTG GAG GAG GCC AAG AAG GTG ATT      192
Trp Pro Glu Leu Val Gly Lys Ser Val Glu Glu Ala Lys Lys Val Ile
    25                  30              35

CTG CAG GAC AAG CCA GAG GCG CAA ATC ATA GTT CTG CCG GTG GGT ACC      240
Leu Gln Asp Lys Pro Glu Ala Gln Ile Ile Val Leu Pro Val Gly Thr
40                  45              50                  55

ATT GTG ACC ATG GAA TAT CGG ATC GAT CGC GTC CGC CTC TTT GTC GAT      288
Ile Val Thr Met Glu Tyr Arg Ile Asp Arg Val Arg Leu Phe Val Asp
                60                  65              70

AAA CTC GAC AAC ATT GCC CAG GTC CCT AGG GTC GGC TAGTGATCTA           334
Lys Leu Asp Asn Ile Ala Gln Val Pro Arg Val Gly
                75                  80

GA                                                                   336
```

(2) INFORMATION FOR SEQ ID NO:4:

    (i) SEQUENCE CHARACTERISTICS:
        (A) LENGTH: 105 amino acids

13

(B)  TYPE: amino acid
(D)  TOPOLOGY: linear

(ii) MOLECULE TYPE: protein

(xi) SEQUENCE DESCRIPTION: SEQ ID NO:4:

```
Met Arg Ser Ser Leu Val Leu Phe Phe Val Ser Ala Trp Thr Ala Leu
-22     -20             -15             -10

Ala Ser Pro Ile Arg Arg Ser Ser Val Glu Lys Lys Pro Glu Gly Val
    -5              1               5                   10

Asn Thr Gly Ala Gly Asp Arg His Asn Leu Lys Thr Glu Trp Pro Glu
            15              20              25

Leu Val Gly Lys Ser Val Glu Glu Ala Lys Lys Val Ile Leu Gln Asp
            30              35              40

Lys Pro Glu Ala Gln Ile Ile Val Leu Pro Val Gly Thr Ile Val Thr
        45              50              55

Met Glu Tyr Arg Ile Asp Arg Val Arg Leu Phe Val Asp Lys Leu Asp
        60              65              70

Asn Ile Ala Gln Val Pro Arg Val Gly
    75              80
```

## Description

### TECHNICAL FIELD

This invention relates to an improved detergent composition comprising a protease (particularly a subtilisin) and a reversible protease inhibitor of peptide or protein type, to a detergent additive comprising such a protease and inhibitor and to a method for stabilizing a protease.

The invention also relates to novel modified subtilisin inhibitors for use in said detergent, to a recombinant DNA molecule comprising a nucleotide sequence coding for the modified subtilisin inhibitor, to a transformed host organism comprising the DNA and to a method of producing the modified inhibitor.

### BACKGROUND ART

Proteases, especially subtilisins, are widely used as ingredients in commercial detergents. A major problem in formulating protease-containing detergents, especially liquid detergents, is that of ensuring enzyme stability during storage.

The prior art has dealt extensively with improving the storage stability. As an example, JP-A 62-269689 demonstrates improvement of the stability of a protease (e.g. a subtilisin) in a liquid detergent by incorporation of a protease inhibitor of protein type. As stated in said publication, the protease inhibitor should ideally show essentially no inhibiting effect under dilute washing conditions, i.e. when the detergent is in use.

### STATEMENT OF THE INVENTION

We have found that in the known detergents containing protease and inhibitor, the protease is almost totally inhibited under dilute washing conditions. We have also found that by a suitable choice of inhibitor for a given protease, it is possible to essentially avoid inhibition at the dilute conditions of washing, while still achieving effective enzyme stabilization in the detergent during storage.

We have also found that subtilisin inhibitors with this improved performance can be derived from known inhibitors by substituting certain amino acids. The novel inhibitors can be produced by known genetic engineering methods.

Accordingly, the invention provides a detergent composition comprising a protease and a reversible protease inhibitor of peptide or protein type, characterized in that the ratio of the dissociation constant to the protease concentration is in the range from 0.006 to 6, or that the dissociation constant is in the range from 0.05 to 50 $\mu$M. The invention also provides a detergent additive comprising protease in the form of a stabilized liquid or a non-dusting granulate, characterized by further comprising a reversible protease inhibitor of peptide or protein type having an dissociation constant in the above range. Further, the invention provides a method for stabilizing a protease by incorporation of a protease inhibitor as described.

Another aspect of the invention provides a detergent composition and a detergent additive comprising a subtilisin, characterized by further comprising a modified subtilisin inhibitor of Family VI having one or more of the following amino acid substitutions at the indicated position:

P6: Ala, Glu or Lys,
P5: Gly, Val, Leu or Pro
P4: Val, Pro, Trp, Ser, Glu or Arg,
P3: Tyr, Glu, Ala, Arg, Pro, Ser, Lys or Trp,
P2: Ser, Lys, Arg, Pro, Glu, Val, Tyr, Trp or Ala,
P1: Arg, Tyr, Pro, Trp, Glu, Val, Ser, Lys or Ala,
P'1: Gln, Ser, Thr, Ile, Lys, or Pro,
P'2: Val, Glu, Arg, Pro or Trp,
P'3: Glu, Gln, Asn, Val, Phe or Tyr.

The invention also provides a modified subtilisin inhibitor of family VI, as defined above, excluding:
Eglin B and C substituted with
Ser or Pro at position 44 (P2),
Leu, Arg, Phe, Tyr at 45 (P1) or
Glu, Ser or Thr at 46 (P'1),
Eglin C substituted with Arg45,Ser46 and
CI-2 substituted with Tyr, Ala or Lys at 59 (P1).

2

Further, the invention provides a recombinant DNA molecule comprising a nucleotide sequence coding for a modified subtilisin inhibitor as defined above, a transformed host organism comprising this DNA and a method of producing the modified inhibitor comprising cultivation of this transformed host organism.

Modified subtilisin inhibitors of family VI are known (EP 332,576, C. Langstaff et al., *Biochemistry*, **1990**, 29, 7339-7347), but their use in detergents and the resulting advantages have not been disclosed or suggested.

## DETAILED DESCRIPTION OF THE INVENTION

Protease

The protease used in the invention is preferably of microbial origin. It may be a serine protease, preferably an alkaline microbial protease or a trypsin-like protease. Examples of alkaline proteases are subtilisins, especially those derived from *Bacillus*, e.g. subtilisin Novo, subtilisin Carlsberg, subtilisin 309, subtilisin 147 and subtilisin 168 (both described in WO 89/06279) and mutant subtilisins such as those described in WO 89/06279 and DK 0541/90. Examples of commercial *Bacillus* subtilisins are Alcalase®, Savinase® and Esperase®, products of Novo Nordisk A/S. Examples of trypsin-like proteases are trypsin (e.g. of porcine or bovine origin) and the *Fusarium* protease described in WO 89/06270.

The amount of protease in the detergent will typically be 0.2-40 $\mu$M, especially 1-20 $\mu$M (generally 5-1000 mg/l, especially 20-500 mg/l) as pure enzyme protein.

Inhibitor

According to the invention, the inhibitor is chosen for a given detergent (protease type and concentration etc.) so that the dissociation constant ($K_d$) is high enough to allow adequate release of protease when the detergent is diluted with water, yet the dissociation constant is low enough to allow efficient inhibition in the concentrated detergent during storage. $K_d$ is commonly defined for a given protease and a given inhibitor in a given system as the equilibrium constant

$$K_d = [E] * [I] / [EI]$$

where the square brackets indicate molar concentration of free enzyme (E), free inhibitor (I) and enzyme-inhibitor complex (EI), respectively.

The ratio of the dissociation constant to the protease concentration is preferably from 0.06 to 6. The dissociation constant is preferably from 1 to 10 $\mu$M (i.e. $10^{-6}$-$10^{-5}$ M).

The desired $K_d$ is achieved by suitable selection of protease and inhibitor. The inhibitor is one of the novel modified inhibitors provided by the invention. For a general description of known inhibitors, see e.g. Lakowski, Jr. & Kato, Ann. Rev. Biochem. 49:593-626 (1980) and S. Murao et al., in Protein Protease Inhibitor - The Case of Streptomyces Subtilisin Inhibitor (1985) at pp. 1-14.

The amount of inhibitor is preferably such that the molar ratio of inhibitor reactive site to protease active site is above 0.6, preferably 1-10.

Novel inhibitor

The novel inhibitors provided by the invention are derived from the known inhibitors of Family VI, described in the above references, e.g. from barley subtilisin inhibitor CI-1 or CI-2, potato subtilisin inhibitor (PSI) Eglin B or C, tomato subtilisin inhibitor or *Vicia* subtilisin inhibitor (VSI).

Inhibitors of this family are known to strongly inhibit the subtilisins commonly used in detergents, with inhibitor dissociation constants generally below $10^{-10}$ M. We have found that by using these inhibitors to stabilize a protease in a detergent, the protease is so strongly bound that very little protease activity is released when the detergent is diluted for use in washing, and the protease remains almost completely inactive. We have therefore realized a need for a modified inhibitor with weaker binding to the protease.

The following shows a comparison of the sequences in the binding region of some family VI inhibitors. Starting from the reactive site, amino acid positions are numbered P1, P2 etc. in the direction of the N-terminal and P'1, P'2 etc towards the C-terminal.

| Inhibitor | P6 | P5 | P4 | P3 | P2 | P1 | P'1 | P'2 | P'3 |
|---|---|---|---|---|---|---|---|---|---|
| CI-1 | Asp | Ala | Met | Val | His | Leu | Asn | Phe | Asp |
| CI-2 | Gly | Thr | Ile | Val | Thr | Met | Glu | Tyr | Arg |
| PSI | Gly | Ser | Pro | Val | Thr | Met | Asp | Phe | Arg |
| Eglin C | Gly | Ser | Pro | Val | Thr | Leu | Asp | Leu | Arg |
| Eglin B | Gly | Ser | Pro | Val | Thr | Leu | Asp | Leu | Arg |
| TSI-1 | Gly | Ser | Pro | Ile | Thr | Leu | Asp | Tyr | Leu |
| VSI | Gly | Ser | Phe | Val | Thr | Ala | Asp | Tyr | Lys |
| Variation | | | | | | | | | |
| | Asp | Ala | Met | Val | His | Leu | Asn | Phe | Asp |
| | Gly | Thr | Ile | Ile | Thr | Met | Glu | Tyr | Arg |
| | | Ser | Pro | | | Ala | Asp | Leu | Leu |
| | | | Phe | | | | | | Lys |
| Modifications according to invention | | | | | | | | | |
| | Ala | Gly | Val | Tyr | Ser | Arg | Gln | Val | Glu |
| | Glu | Val | Pro | Glu | Lys | Glu | Ser | Glu | Gln |
| | Lys | Leu | Trp | Ala | Arg | Val | Pro | Arg | Asn |
| | | Pro | Ser | Arg | Pro | Ser | Ile | Pro | Val |
| | | | Glu | Pro | Glu | Lys | Thr | Trp | Phe |
| | | | Arg | Ser | Val | Tyr | Lys | | Tyr |
| | | | | Lys | Tyr | Pro | | | |
| | | | | Trp | Trp | Ala | | | |
| | | | | | Ala | Trp | | | |

It appears that the inhibitors show a marked homology in the binding region. We have now found that the protease-inhibitor binding can be suitably weakened by substituting one or more of these amino acids, e.g. with one that is not represented at that position, i.e. with one that has a different side chain length and/or is differently charged from those represented. The modified inhibitors are resistant to hydrolysis by the protease.

A preferred inhibitor is CI-2 substituted with Arg, Pro or Glu at position P3, Lys or Arg at P2, and/or Glu, Arg or Pro at P1. Another preferred inhibitor is PSI substituted with Tyr at P3, Lys or Arg at P2, Arg, Tyr or His at P1 and/or Trp at P'1.

The novel inhibitors may be produced by known genetic engineering techniques. Briefly, a DNA sequence (cDNA or a synthetic gene) encoding a known inhibitor is subjected to mutagenesis in order to replace the codon(s) for the amino acid(s) to be substituted with a new codon (codons) for the desired amino acid substitution(s). This may preferably be carried out by oligonucleotide-directed site-specific mutagenesis in bacteriophage M13 vectors (e.g. M.J. Zoller and M. Smith, Meth. Enzymol. 100 (1983) 468-500), in double-stranded DNA vectors (e.g. Y. Morinaga et al., Biotechnology (July 1984) 636-639), or by the polymerase chain reaction (PCR) (e.g. R. Higuchi, Nucl. Acids. Res. 16 (1988) 7351-7367).

The mutant gene is subsequently expressed in a suitable host strain. Suitable hosts are bacteria (e.g. strains of *Escherichia coli* or *Bacillus*), fungi (e.g. strains of *Saccharomyces cerevisiae* or filamentous fungi like *Aspergillus*), plants such as tomato or potato or established human or animal cell lines. To accomplish expression, the mutant gene has to be inserted in an expression plasmid with promoter and terminator DNA elements for the formation of translatable mutant inhibitor mRNA *in vivo*. The plasmid is introduced into the host by genetic transformation. The choice of expression plasmid is dependent on the type of host strain used. The expression of the mutant inhibitor may be done intracellularly or extracellularly. In the latter case, the DNA sequence coding for the mutant inhibitor is fused in frame to a DNA sequence encoding a suitable peptide signaling secretion. The secretion signal should preferably be cleaved off *in vivo*, resulting in secretion of the mature mutant inhibitor into the growth medium.

Various species of Bacilli, including *Bacillus alkalophilus*, *B. amyloliquefaciens*, *B. brevis*, *B. lentus*, *B. licheniformis*, *B. megaterium*, *B. stearothermophilus*, and *B. subtilis*, are known to secrete proteins efficiently. In many cases this has also been shown to be the case for heterologous proteins. Since expression of a secreted protease inhibitor has the potential advantage of facilitating purification, it is obviously interesting to attempt to express the inhibitor as a secreted product from a *Bacillus* strain. This could for instance be accomplished by combining the structural part of the inhibitor with the promoter and signal peptide of a well expressed and secreted *Bacillus* enzyme as for instance the maltogenic amylase from *B. stearothermophilus* (Diderichsen, B. and Christiansen, L. Cloning of a maltogenic alpha-amylase from *Bacillus stearothermophilus*, FEMS Microbiol. Lett. 56:53-60. 1988) or the alpha- amylase from *B. licheniformis* (Jørgensen, P.L., C.K. Hansen, G.B. Poulsen and B. Diderichsen. *In vivo* genetic engineering: Homologous recombination as a tool for plasmid construction, GENE 96: 37-41, 1990). This may be accomplished in many ways as known by people skilled in the art. One way is to use *in vivo* genetic engineering (Jørgensen et al. 1990, op. cit.). The advantage of this method is that it easily generates a perfect fusion between signal peptide and mature inhibitor which according to well documented rules for signal peptide processing would be expected to give the correct N-terminal amino acid residue of the inhibitor.

In one method of producing barley CI-2A inhibitor and variants hereof, a filamentous fungus is used as the host organism. The filamentous fungus host organism may conveniently be one which has previously been used as a host for producing recombinant proteins, e.g. a strain of *Aspergillus sp.*, such as *A. niger*, *A. nidulans* or *A. oryzae*. The use of *A. oryzae* in the production of recombinant proteins is extensively described in, e.g. EP 238 023.

For expression of CI-2A inhibitor and variants in *Aspergillus*, the DNA sequence encoding the protease inhibitor is preceeded by a promoter. The promoter may be any DNA sequence exhibiting a strong transcriptional activity in Aspergillus and may be derived from a gene encoding an extracellular or intracellular protein such as an amylase, a glucoamylase, a protease, a lipase, a cellulase or a glycolytic enzyme.

Examples of suitable promoters are those derived from the gene encoding *A. oryzae* TAKA amylase, *Rhizomucor miehei* aspartic proteinase, *A. niger* neutral alfa-amylase, *A. niger* acid stable alfa-amylase, *A. niger* glucoamylase, *Rhizomucor miehei* lipase, *A. oryzae* alkaline protease or *A. oryzae* triose phosphate isomerase.

In particular when the host organism is *A. oryzae*, a preferred promoter for use in the process of the present invention is the *A. oryzae* TAKA amylase promoter as it exhibits a strong transcriptional activity in *A. oryzae*. The sequence of the TAKA amylase promoter appears from EP 238 023.

Termination and polyadenylation sequences may suitably be derived from the same sources as the promoter.

To ensure secretion of the inhibitor or variants hereof from the host cell, the DNA sequence encoding the inhibitor may be preceded by a signal sequence which may be a naturally occurring signal sequence or a functional part thereof or a synthetic sequence providing secretion of the protein from the cell. In particular, the signal sequence may be derived from a gene encoding an *Aspergillus sp.* amylase or glucoamylase, a gene encoding a *Rhizomucor miehei* lipase or proteinase, or a gene encoding a *Humicola* cellulase, xylanase or lipase.

Detergent

The detergent of the invention may be in any convenient form, e.g. powder, granules or liquid. A liquid detergent may be aqueous, typically containing 20-70% water and 0-20% organic solvent.

The detergent comprises surfactant which may be anionic, non-ionic, cationic, amphoteric or a mixture of these types. The detergent will usually contain 5-30% anionic surfactant such as linear alkyl benzene sulphonate (LAS), alpha-olefin sulphonate (AOS), alcohol ethoxy sulphate (AES) or soap. It may also contain 3-20% anionic surfactant such as nonyl phenol ethoxylate or alcohol ethoxylate.

The pH (measured in aqueous detergent solution) will usually be neutral or alkaline, e.g. 7-10. The detergent may contain 1-40% of a detergent builder such as zeolite, phosphate, phosphonate, citrate, NTA, EDTA or DTPA, or it may be unbuilt (i.e. essentially free of a detergent builder). It may also contain other conventional detergent ingredients, e.g. fabric conditioners, foam boosters, bactericides, optical brighteners and perfumes.

Specific examples of detergents according to the invention may be obtained from the compositions disclosed in WO 89/04361, DK 5111/89 or PCT/DK91/00243 by incorporating protease and inhibitor according to the invention. PCT/DK91/00243 is incorporated herein by reference.

The invention is particularly applicable to the formulation of liquid detergents with pronounced enzyme stability problems, e.g those containing oxidizing agents. Such detergents typically contain 1-40%, especially 5-20% oxidizing agent. They may be granular detergents containing granules of a perborate or percarbonate and separate granules containing enzyme and inhibitor according to the invention, or they may be aqueous or non-aqueous liquid detergents containing hydrogen peroxide, a perborate or a percarbonate (see e.g. EP 378,261, EP 378,262, EP 294,904, EP 368,575).

Detergent additive

The protease and inhibitor may be included in the detergent of the invention by separate addition or by adding the combined additive provided by the invention. The additive will usually contain 0.2-8 mM protease (0.5-20%) and have an inhibitor/protease ratio as described above.

The detergent additive may be in liquid form for incorporation in a liquid detergent. A liquid additive may contain 20-90% propylene glycol; 0.5-3% (as Ca) of a soluble calcium salt; 0-10% glycerol; minor amounts of short-chain fatty acids and carbohydrate; and water up to 100%.

**EXAMPLE 1**

Expression of barley subtilisin inhibitor CI-2A in *Saccharomyces cerevisiae*

The barley subtilisin inhibitor and variants hereof according to the invention can be produced biosynthetically in a yeast host expressing a DNA sequence encoding the inhibitor.

To achieve secretion to the growth medium, the DNA sequence encoding the inhibitor can be fused to another DNA-sequence encoding a signal peptide functional in yeast. An example hereof is the *Saccharomyces cerevisiae* MF-alfa-1 leader sequence (Kurjan & Herskowitz, Cell 30, 933-943 (1982). A preferred construction uses the DNA sequence encoding the entire 85 aminoacid MF-alfa-1 leader sequence including the dibasic site LysArg. In that way, an efficient secretion of CI-2A inhibitor with the correct N-terminal is achieved.

Plasmid construction

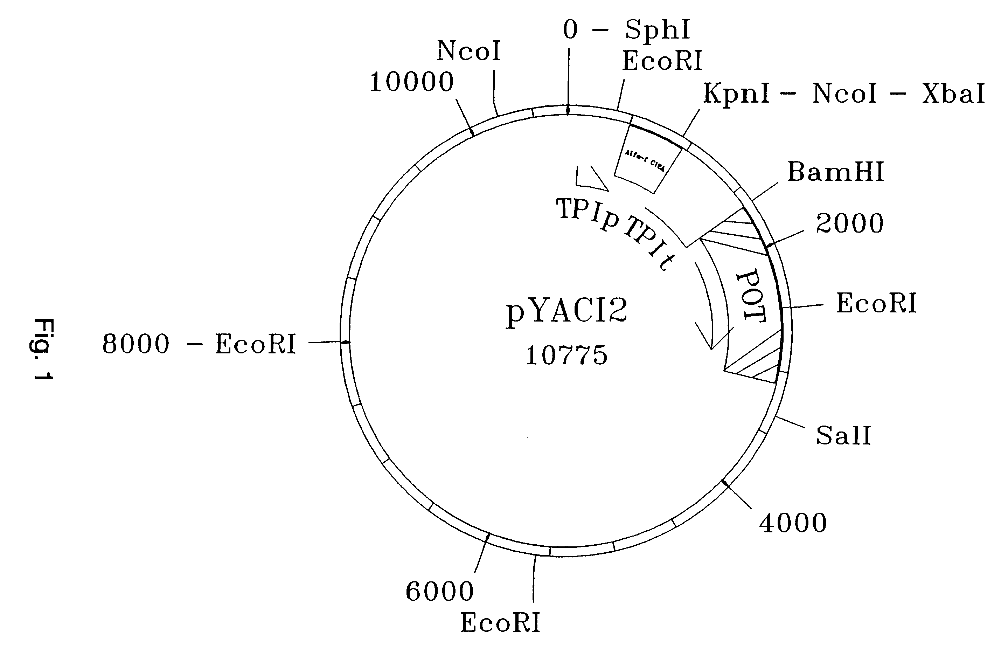

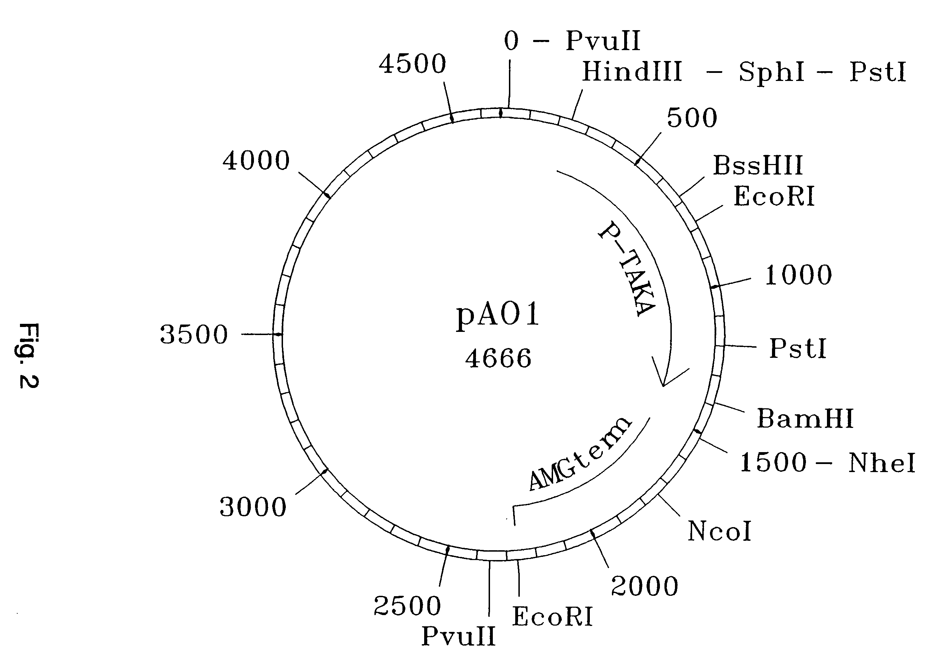

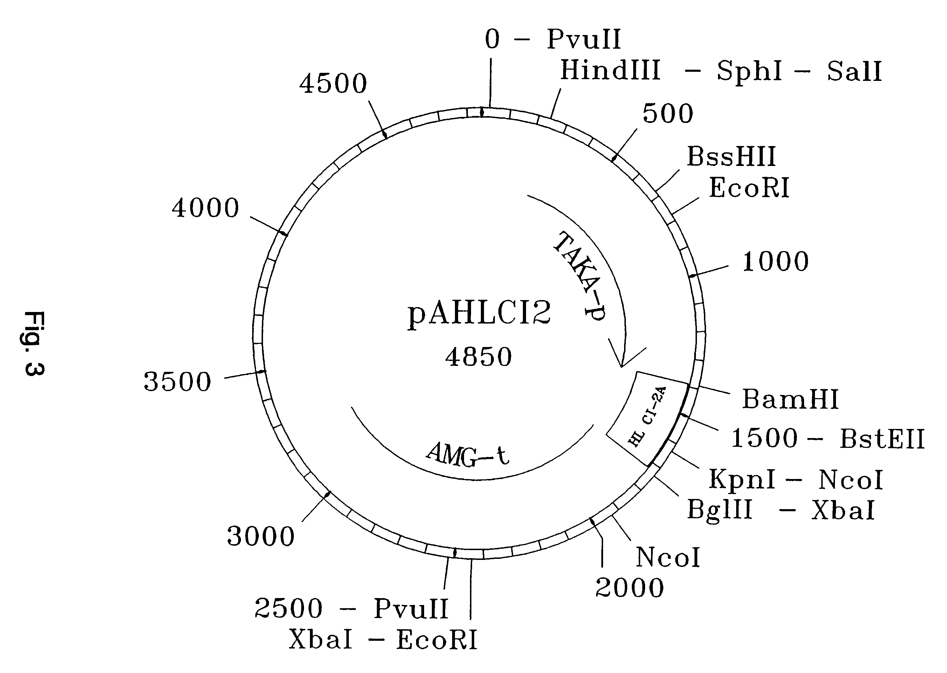

All expression plasmids are of the C-POT type. Such plasmids are described in EP patent application No. 85303702.6 and are characterized in containing the *S. pombe* triose phosphate isomerase gene (POT) for the purpose of plasmid stabilization. A plasmid containing the POT-gene is available from a deposited *E.coli* strain (ATCC 39685). The plasmids furthermore contain the *S. cerevisiae* triose phosphate isomerase promoter and terminater ($P_{TPI}$ and $T_{TPI}$).They are identical to pLaC200 described in the patent application WO 89/02463, except for the region defined by the EcoRI/XbaI restriction fragment encoding a signal/leader/insulin precursor sequence. In this application, the region is replaced by a fragment encoding MF-alfa-1 leader fused to the inhibitor sequence. The sequence of the fragment is shown in Sequence Listing ID No. 1 (P1 is located at Met 59). The isolation of the barley CI-2A subtilisin inhibitor cDNA is described by Williamson et al. Eur. J. Biochem. 165, 99-106 (1987). Cloning of the MF-alfa-1 leader is described by Kurjan & Herskowitz (reference given above). Modifications and assembly of the two sequences were carried out using entirely standard techniques. In particular, the KpnI and ClaI restriction sites at positions 495 and 519, respectively, were generated by introducing silent mutations into the inhibitor gene. This was done carrying out *in vitro* mutagenesis on the inhibitor gene. A map of the expression plasmid pYACI2 is shown in Figure 2.

Introduction of mutations into the inhibitor gene

Mutant CI-2A genes were generated using PCR mutagenesis, which was carried out as follows: A primer carrying the mutation flanked by homologous sequences and carrying the introduced KpnI-site was used together with another primer homologous to sequences in the $T_{TPI}$ region in a PCR amplification reaction. In that way, fragments were generated, which contained the desired mutations. The ends were trimmed with the restriction enzymes KpnI and XbaI, purified on agarose gels, and cloned into pYACI2 previously digested with the same restriction enzymes. The presence of the mutation was verifyed by DNA sequencing. The primers used are listed below.

6

| Mutation | Primer sequence |
|----------|-----------------|
| P4 Ile -> Pro | 5'-CCGGTGGGTACCCCAGTGACCATGGAA-3' |
| P4 Ile -> Trp | 5'-CCGGTGGGTACCTGGGTGACCATGGAA-3' |
| P3 Val -> Glu | 5'-GTGGGTACCATTGAAACCATGGAATAT-3' |
| P3 Val -> Ala | 5'-GTGGGTACCATTGCTACCATGGAATAT-3' |
| P3 Val -> Arg | 5'-GTGGGTACCATTAGAACCATGGAATAT-3' |
| P3 Val -> Tyr | 5'-GTGGGTACCATTTACACCATGGAATAT-3' |
| P3 Val -> Pro | 5'-GTGGGTACCATTCCAACCATGAAGTAT-3' |
| P2 Thr -> Glu | 5'-GTGGGTACCATTGTGGAAATGGAATATCGG-3' |
| P2 Thr -> Val | 5'-GTGGGTACCATTGTGGTTATGGAATATCGG-3' |
| P2 Thr -> Arg | 5'-GTGGGTACCATTGTGAGAATGGAATATCGG-3' |
| P2 Thr -> Tyr | 5'-GTGGGTACCATTGTGTACATGGAATATCGG-3' |
| P2 Thr -> Pro | 5'-GTGGGTACCATTGTGCCAATGGAATATCGG-3' |
| P1 Met -> Glu | 5'-GTGGGTACCATTGTGACCGAAGAATATCGGATC-3' |
| P1 Met -> Val | 5'-GTGGGTACCATTGTGACCGTTGAATATCGGATC-3' |
| P1 Met -> Arg | 5'-GTGGGTACCATTGTGACCAGAGAATATCGGATC-3' |
| P1 Met -> Tyr | 5'-GTGGGTACCATTGTGACCTACGAATATCGGATC-3' |
| P1 Met -> Pro | 5'-GTGGGTACCATTGTGACCCCAGAATATCGGATC-3' |

The sequence of the other primer used for generation of PCR products, and which has homology to the $T_{TPI}$ terminater region is: 5'-TTAAGTGGCTCAGAATG-3'

Expression of mutant CI-2A inhibitors in yeast

Plasmids prepared as described above were transformed into a *S. cerevisiae* strain carrying deletions in the TPI gene by selecting for growth on glucose.

The transformed yeast strains were grown on YPD medium (Sherman, F. et al., Methods in Yeast Genetics, Cold Spring Harbor Laboratory 1981). 100 ml medium in shake-flasks was inoculated with individual transformants and shaken at 30°C for approx. 48 hours after which the inhibitor could be purified from the medium.

**EXAMPLE 2**

Expression of barley chymotrypsin inhibitor CI-2A in *Aspergillus oryzae*

Plasmid constructions

Cloning and expression of *Humicola lanuginosa* lipase in *Aspergillus oryzae* is described in EP 305,216. The same host/vector system can be used for expression and secretion of barley chymotrypsin inhibitor CI-2A. The lipase expression plasmid is termed p960 and makes use of the *A. oryzae* TAKA amylase promoter for driving the transcription and the *Aspergillus niger* glucoamylase transcription terminater.

The plasmid p960 was slightly modified in order to obtain a vector for cloning the inhibitor gene. p960 was digested with NruI and BamHI restriction enzymes. Between these two sites the BamHI/NheI fragment from pBR322, in which the NheI-site was filled in with Klenow polymerase and dNTP's, was cloned, thereby creating plasmid pAO1 (Fig. 3) which contains unique BamHI and NheI sites facilitating cloning of BamHI/XbaI fragments.

A BamHI/AvaI linker with the sequence

```
BamHI
GATCCACCATGAGGAGCTCCCTTGTGCTGTTCTTTGTCTCTGCGTGGACGGCCTTGGCCAGTC
     GTGGTACTCCTCGAGGGAACACGACAAGAAACAGAGACGCACCTGCCGGAACCGGTCAG
          MetArgSerSerLeuValLeuPhePheValSerAlaTrpThrAlaLeuAlaSerP


CTATTCGTCGAAGCTCAGTGGAGAAGAAGC      AvaI
GATAAGCAGCTTCGAGTCACCTCTTCTTCGGGCT
roIleArgArgSerSerValGluLysLysPro
```

encoding the *Humicola lanuginosa* lipase pre-pro sequence and part of the CI-2A inhibitor was combined with the AvaI/XbaI fragment from pYACl2 and cloned into pAO1 digested with BamHI and NheI, thereby creating the expression plasmid pAHLCI2 (Fig. 4). The sequence of the BamHI/XbaI insert is shown in Sequence Listing ID No. 3 (P1 at Met 59).

Transformation of *Aspergillus oryzae* (general procedure)

100 ml of YPD (Sherman et al., Methods in Yeast Genetics, Cold Spring Harbor Laboratory, 1981) was inoculated with spores of *A. oryzae* and incubated with shaking for about 24 hours. The mycelium was harvested by filtration through miracloth and washed with 200 ml of 0.6 M $MgSO_4$. The mycelium was suspended in 15 ml of 1.2 M $MgSO_4$, 10 mM $NaH_2PO_4$, pH = 5.8. The suspension was cooled on ice and 1 ml of buffer containing 120 mg of Novozym® 234, batch 1687 was added. After 5 min., 1 ml of 12 mg/ml BSA (Sigma type H25) was added and incubation with gentle agitation continued for 1.5 - 2.5 hours at 37°C until a large number of protoplasts was visible in a sample inspected under the microscope.

The suspension was filtered through miracloth, the filtrate transferred to a sterile tube and overlayed with 5 ml of 0.6 M sorbitol, 100 mM Tris-HCl, pH = 7.0. Centrifugation was performed for 15 min. at 1000 g and the protoplasts were collected from the top of the $MgSO_4$ cushion. 2 volumes of STC (1.2 M sorbitol, 10 mM Tris-HCl, pH = 7.5, 10 mM $CaCl_2$) were added to the protoplast suspension and the mixture was centrifugated for 5 min. at 1000 g. The protoplast pellet was resuspended in 3 ml of STC and repelleted. This was repeated. Finally, the protoplasts were resuspended in 0.2 - 1 ml of STC.

100 $\mu$l of protoplast suspension was mixed with 5 - 25 $\mu$g of p3SR2 (an *A. nidulans* amdS gene carrying plasmid described in Hynes et al., Mol. and Cel. Biol., Vol. 3, No. 8, 1430-1439, Aug. 1983) in 10 $\mu$l of STC. The mixture was left at room temperature for 25 min. 0.2 ml of 60% PEG 4000 (BDH 29576), 10 mM $CaCl_2$ and 10 mM Tris-HCl, pH = 7.5 was added and carefully mixed (twice) and finally 0.85 ml of the same solution was added and carefully mixed. The mixture was left at room temperature for 25 min., spun at 2.500 g for 15 min. and the pellet was resuspended in 2 ml of 1.2 M sorbitol. After one more sedimentation the protoplasts were spread on minimal plates (Cove, Biochem. Biophys. Acta 113 (1966) 51-56) containing 1.0 M sucrose, pH = 7.0, 10 mM acetamide as nitrogen source and 20 mM CsCl to inhibit background growth. After incubation for 4 - 7 days at 37°C spores were picked, suspended in sterile water and spread for single colonies. This procedure was repeated and spores of a single colony after the second reisolation were stored as a defined transformant.

Expression of the barley inhibitor CI-2A in *A. oryzae*

pAHLCI2 was transformed into *A. oryzae* IFO 4177 by cotransformation with p3SR2 containing the amdS gene from *A. nidulans* as described above. Protoplasts prepared as described were incubated with a mixture of equal amounts of pAHLCI2 and p3SR2, approximately 5 $\mu$g of each were used. 9 transformants which could use acetamide as sole nitrogen source were reisolated twice. After growth on YPD for three days, culture supernatants were analyzed for inhibitor activity.

**EXAMPLE 3**

Purification of the wild-type CI-2 inhibitor and mutants thereof

Fermentation broths containing either the wild-type CI-2 inhibitor or one of the following CI-2 inhibitor mutants: CI-2(M59P), CI-2(V57E), CI-2(M59E), CI-2(M59R), CI-2(V57R) and CI-2(V57P + E60K), produced as described in example 1, were filtered on a pressure filter (Zeitz K 250-Neu) provided with 0.5% filter aid, and subsequently on a Zeitz EK-1 filter provided with 0.5% filter aid. The filtrate was diluted to a condictivity of <4 mS with 50 mM sodium acetate, pH 4.4, and water, and the pH was adjusted to 4.4. The filtrate was then subjected to chromatography on a S-Sepharose FPLC column using a 50 mM sodium acetate buffer, pH 4.4, and a 50 mM sodium acetate buffer supplemented with 1M NaCl. Elution of the column was performed with a 0-100% gradient of 50 mM sodium acetate buffer supplemented with 1M NaCl.

To effect a change of buffer, the eluate was run through a Sephadex G-25 column into two different buffers: (a) 50 mM glycine-NaOH buffer, pH 9.6, and (b) 50 mM $H_3BO_3$-NaOH buffer, pH 10.2 (buffer (b) being used for basic mutants (CI-2(M59R), CI-2(V57R) and CI-2(V57P + E60K)). The eluate was then subjected to chromatography on a Q-Sepharose column using either buffer (a) or buffer (b), as appropriate. The column was eluted with a gradient of 0-1 M NaCl. Inhibitor-containing fractions were collected and used in the subsequent experiments.

**EXAMPLE 4**

Interaction of protease with inhibitor

The interaction of Alcalase® with wild-type CI-2, CI-2(M59P) and CI-2(V57E), respectively, was studied in a 0.1 M Tris-HCl buffer, pH 8.6, at 25°C, using the synthetic peptide substrates Suc-Ala-Ala-Pro-Phe-pNA and Suc-Ala-Ala-Ala-pNA (both available from Sigma) to determine residual activity after reacting the protease with the inhibitor in amounts from 0 to 1.5 times the protease concentration.

The following dissociation constants were determined using non-linear regression essentially as described in M. Tashiro et al., Agric. Biol. Chem. 55(1), 1991, pp. 265-267.

| Inhibitor | Dissociation constant ($K_d$) |
| --- | --- |
| Wild-type CI-2 | $4 \times 10^{-12}$ M |
| CI-2(M59P) (mutated in P1) | $5 \times 10^{-9}$ M |
| CI-2(V57E) (mutated in P3) | $1 \times 10^{-10}$ M |

The results show that it is possible to change the dissociation constant by several orders of magnitude by single amino acid substitutions in the binding region of the inhibitor.

## Claims

1. A detergent composition comprising a protease and a reversible protease inhibitor of peptide or protein type, characterized in that the inhibitor is a modified subtilisin inhibitor of family VI having one or more of the following amino acid substitutions at the indicated position:
   P6: Ala, Glu or Lys,
   P5: Gly, Val, Leu or Pro,
   P4: Val, Pro, Trp, Ser, Glu or Arg,
   P3: Tyr, Glu, Ala, Arg, Pro, Ser, Lys or Trp,
   P2: Ser, Lys, Arg, Pro, Glu, Val, Tyr, Trp or Ala,
   P1: Arg, Tyr, Pro, Trp, Glu, Val, Ser, Lys or Ala,
   P'1: Gln, Ser, Thr, Ile, Lys or Pro,
   P'2: Val, Glu, Arg, Pro or Trp,
   P'3: Glu, Gln, Asn, Val, Phe or Tyr.

2. A detergent composition according to claim 1, wherein the inhibitor is a modified barley subtilisin inhibitor CI-1 or CI-2, potato subtilisin inhibitor PSI, Eglin B or C, tomato subtilisin inhibitor (TSI) or Vicia subtilisin inhibitor (VSI).

3. A detergent composition according to either of claims 1-2, wherein the protease is a serine protease, preferably an alkaline microbial protease or a trypsin-like protease.

4. A detergent composition according to claim 3, wherein the alkaline microbial protease is a subtilisin.

5. A detergent composition according to claim 4, wherein the subtilisin is derived from Bacillus and is preferably subtilisin Novo, subtilisin Carlsberg, BPN', subtilisin 309, subtilisin 147 or subtilisin 168.

6. An aqueous liquid detergent composition according to any preceding claim.

14

7. A detergent additive comprising protease in the form of a stabilized liquid or a non-dusting granulate, characterized by comprising a reversible protease inhibitor of peptide or protein type wherein the inhibitor is a modified subtilisin inhibitor of family VI having one or more of the following amino acid substitutions at the indicated position:

P6: Ala, Glu or Lys,
P5: Gly, Val, Leu or Pro,
P4: Val, Pro, Trp, Ser, Glu or Arg,
P3: Tyr, Glu, Ala, Arg, Pro, Ser, Lys or Trp,
P2: Ser, Lys, Arg, Pro, Glu, Val, Tyr, Trp or Ala,
P1: Arg, Tyr, Pro, Trp, Glu, Val, Ser, Lys or Ala,
P'1: Gln, Ser, Thr, Ile, Lys or Pro,
P'2: Val, Glu, Arg, Pro or Trp,
P'3: Glu, Gln, Asn, Val, Phe or Tyr.

8. A detergent additive according to claim 7, wherein the inhibitor is a modified barley subtilisin inhibitor CI-1 or CI-2, potato subtilisin inhibitor PSI, Eglin B or C, tomato subtilisin inhibitor (TSI) or Vicia subtilisin inhibitor (VSI).

9. A method for stabilizing a protease by incorporation of a protease inhibitor of peptide or protein type, characterized in that the inhibitor is a modified subtilisin inhibitor of family VI having one or more of the following amino acid substitutions at the indicated position:

P6: Ala, Glu or Lys,
P5: Gly, Val, Leu or Pro,
P4: Val, Pro, Trp, Ser, Glu or Arg,
P3: Tyr, Glu, Ala, Arg, Pro, Ser, Lys or Trp,
P2: Ser, Lys, Arg, Pro, Glu, Val, Tyr, Trp or Ala,
P1: Arg, Tyr, Pro, Trp, Glu, Val, Ser, Lys or Ala,
P'1: Gln, Ser, Thr, Ile, Lys or Pro,
P'2: Val, Glu, Arg, Pro or Trp,
P'3: Glu, Gln, Asn, Val, Phe or Tyr.

10. A method according to claim 9, wherein the inhibitor is a modified barley subtilisin inhibitor CI-1 or CI-2, potato subtilisin inhibitor PSI, Eglin B or C, tomato subtilisin inhibitor (TSI) or Vicia subtilisin inhibitor (VSI).

11. A modified subtilisin inhibitor of family VI having one or more of the following amino acid substitutions at the indicated position:

P6: Ala, Glu or Lys,
P5: Gly, Val, Leu or Pro,
P4: Val, Pro, Trp, Ser, Glu or Arg,
P3: Tyr, Glu, Ala, Arg, Pro, Ser, Lys or Trp,
P2: Ser, Lys, Arg, Pro, Glu, Val, Tyr, Trp or Ala,
P1: Arg, Tyr, Pro, Trp, Glu, Val, Ser, Lys or Ala,
P'1: Gln, Ser, Thr, Ile, Lys or Pro,
P'2: Val, Glu, Arg, Pro or Trp,
P'3: Glu, Gln, Asn, Val, Phe or Tyr.
excluding:
Eglin B and C substituted with
Pro at position 42 (P4),
Ser or Pro at position 44 (P2),
Leu, Arg, Lys, Phe, Trp, Tyr at 45 (P1) or
Glu, Gln, Ser or Thr at 46 (P'1),
Eglin C substituted with Arg45, Ser46,
CI-2 substituted with Tyr, Ala or Lys at 59 (P1) and
CI-1B substituted with Pro in P2 and Asn in P'3.

12. A modified barley subtilisin inhibitor CI-1 or CI-2, potato subtilisin inhibitor PSI, Eglin B or C, tomato subtilisin inhibitor (TSI) or Vicia subtilisin inhibitor (VSI) according to claim 11.

13. A recombinant DNA molecule comprising a nucleotide sequence coding for the modified subtilisin inhibitor of claim 11 or 12.

14. A transformed host organism comprising the DNA of claim 13.

15. A method of producing the inhibitor of Claim 11 or 12, characterized by comprising cultivation of the transformed host organism of claim 14.

**Patentansprüche**

1. Detergenszusammensetzung, enthaltend eine Protease und einen reversiblen Protease-Inhibitor vom Peptid- oder Proteintyp, dadurch gekennzeichnet, daß es sich beim Inhibitor um einen modifizierten Subtilisin-Inhibitor der Familie VI mit einer oder mehreren der folgenden Aminosäuresubstitutionen an der angegebenen Position handelt:
   P6: Ala, Glu or Lys,
   P5: Gly, Val, Leu oder Pro,
   P4: Val, Pro, Trp, Ser, Glu oder Arg,
   P3: Tyr, Glu, Ala, Arg, Pro, Ser, Lys oder Trp,
   P2: Ser, Lys, Arg, Pro, Glu, Val, Tyr, Trp oder Ala,
   P1: Arg, Tyr, Pro, Trp, Glu, Val, Ser, Lys oder Ala,
   P'1: Gln, Ser, Thr, Ile, Lys oder Pro,
   P'2: Val, Glu, Arg, Pro oder Trp,
   P'3: Glu, Gln, Asn, Val, Phe oder Tyr.

2. Detergenszusammensetzung nach Anspruch 1, wobei es sich beim Inhibitor um einen modifizierten Gerste-Subtilisin-Inhibitor CI-1 oder CI-2, Kartoffel-Subtilisin-Inhibitor PSI, Eglin B oder C, Tomaten-Subtilisin-Inhibitor (TSI) oder *Vicia*-Subtilisin-Inhibitor (VSI) handelt.

3. Detergenszusammensetzung nach einem der Ansprüche 1-2, wobei es sich bei der Protease um eine Serinprotease, vorzugsweise eine alkalische mikrobielle Protease oder eine trypsinähnliche Protease, handelt.

4. Detergenszusammensetzung nach Anspruch 3, wobei es sich bei der alkalischen mikrobiellen Protease um ein Subtilisin handelt.

5. Detergenszusammensetzung nach Anspruch 4, wobei das Subtilisin von Bacillus abgeleitet ist und es sich vorzugsweise um Subtilisin-Novo, Subtilisin-Carlsberg, BPN', Subtilisin 309, Subtilisin 147 oder Subtilisin 168 handelt.

6. Wäßrige flüssige Detergenszusammensetzung nach einem der vorstehenden Ansprüche.

7. Detergens-Additiv, enthaltend Protease in Form eines stabilisierten, flüssigen oder nicht-staubenden Granulats, gekennzeichnet durch einen Gehalt an einem reversiblen Protease-Inhibitor vom Peptid- oder Proteintyp, wobei es sich beim Inhibitor um einen modifizierten Subtilisin-Inhibitor der Familie VI mit einer oder mehreren der folgenden Aminosäuresubstitutionen an der angegebenen Position handelt:
   P6: Ala, Glu or Lys,
   P5: Gly, Val, Leu oder Pro,
   P4: Val, Pro, Trp, Ser, Glu oder Arg,
   P3: Tyr, Glu, Ala, Arg, Pro, Ser, Lys oder Trp,
   P2: Ser, Lys, Arg, Pro, Glu, Val, Tyr, Trp oder Ala,
   P1: Arg, Tyr, Pro, Trp, Glu, Val, Ser, Lys oder Ala,
   P'1: Gln, Ser, Thr, Ile, Lys oder Pro,
   P'2: Val, Glu, Arg, Pro oder Trp,
   P'3: Glu, Gln, Asn, Val, Phe oder Tyr.

8. Detergens-Additiv nach Anspruch 7, wobei es sich beim Inhibitor um einen modifizierten Gerste-Subtilisin-Inhibitor CI-1 oder CI-2, Kartoffel-Subtilisin-Inhibitor PSI, Eglin B oder C, Tomaten-Subtilisin-Inhibitor (TSI) oder *Vicia*-Subtilisin-Inhibitor (VSI) handelt.

9. Verfahren zum Stabilisieren einer Protease durch Einverleiben eines Protease-Inhibitors vom Peptid- oder Proteintyp, dadurch gekennzeichnet, daß es sich beim Inhibitor um einen modifizierten Subtilisin-Inhibitor der Familie VI mit einer oder mehreren der folgenden Aminosäuresubstitutionen an der angegebenen Position handelt:

P6: Ala, Glu or Lys,
P5: Gly, Val, Leu oder Pro,
P4: Val, Pro, Trp, Ser, Glu oder Arg,
P3: Tyr, Glu, Ala, Arg, Pro, Ser, Lys oder Trp,
P2: Ser, Lys, Arg, Pro, Glu, Val, Tyr, Trp oder Ala,
P1: Arg, Tyr, Pro, Trp, Glu, Val, Ser, Lys oder Ala,
P'1: Gln, Ser, Thr, Ile, Lys oder Pro,
P'2: Val, Glu, Arg, Pro oder Trp,
P'3: Glu, Gln, Asn, Val, Phe oder Tyr.

10. Verfahren nach Anspruch 9, wobei es sich beim Inhibitor um einen modifizierten Gerste-Subtilisin-Inhibitor CI-1 oder CI-2, Kartoffel-Subtilisin-Inhibitor PSI, Eglin B oder C, Tomaten-Subtilisin-Inhibitor (TSI) oder *Vicia*-Subtilisin-Inhibitor (VSI) handelt.

11. Modifizierter Subtilisin-Inhibitor der Familie VI mit einer oder mehreren der folgenden Aminosäuresubstitutionen an der angegebenen Position:

P6: Ala, Glu or Lys,
P5: Gly, Val, Leu oder Pro,
P4: Val, Pro, Trp, Ser, Glu oder Arg,
P3: Tyr, Glu, Ala, Arg, Pro, Ser, Lys oder Trp,
P2: Ser, Lys, Arg, Pro, Glu, Val, Tyr, Trp oder Ala,
P1: Arg, Tyr, Pro, Trp, Glu, Val, Ser, Lys oder Ala,
P'1: Gln, Ser, Thr, Ile, Lys oder Pro,
P'2: Val, Glu, Arg, Pro oder Trp,
P'3: Glu, Gln, Asn, Val, Phe oder Tyr,
ausgenommen:
Eglin B und C, substituiert durch
Pro in Position 42 (P4)
Ser oder Pro in Position 44 (P2),
Leu, Arg, Lys, Phe, Trp, Tyr in 45 (P1) oder
Glu, Gln, Ser oder Thr in 46 (P'1),
Eglin C, substituiert durch Arg45, Ser 46,
CI-2, substituiert durch Tyr, Ala oder Lys in 59 (P1) und
CI-1B, substituiert durch Pro in P2 und Asn in P'3.

12. Modifizierter Gerste-Subtilisin-Inhibitor CI-1 oder CI-2, Kartoffel-Subtilisin-Inhibitor PSI, Eglin B oder C, Tomaten-Subtilisin-Inhibitor (TSI) oder *Vicia*-Subtilisin-Inhibitor (VSI) nach Anspruch 11.

13. Rekombinantes DNA-Molekül, enthaltend eine Nukleotidsequenz, die für den modifizierten Subtilisin-Inhibitor von Anspruch 11 oder 12 kodiert.

14. Transformierter Wirtsorganismus, enthaltend die DNA von Anspruch 13.

15. Verfahren zur Erzeugung des Inhibitors von Anspruch 11 oder 12, gekennzeichnet durch Züchtung des transformierten Wirtsorganismus von Anspruch 14.

**Revendications**

1. Composition de détergent comportant une protéase et un inhibiteur réversible de protéase de type peptidique ou protéique, caractérisée en ce que l'inhibiteur est un inhibiteur de la subtilisine modifié de la famille VI ayant une ou plusieurs des substitutions d'acides aminées suivantes au niveau de la position indiquée :

P6 : Ala, Glu ou Lys
P5 : Gly, Val, Leu ou Pro

P4 : Val, Pro, Trp, Ser, Glu ou Arg

P3 : Tyr, Glu, Ala, Arg, Pro, Ser, Lys ou Trp,

P2 : Ser, Lys, Arg, Pro, Glu, Val, Tyr, Trp ou Ala

P1 : Arg, Tyr, Pro, Trp, Glu, Val, Ser, Lys ou Ala

P'1 : Gln, Ser, Thr, Ile, Lys ou Pro,

P'2 : Val, Glu, Arg, Pro ou Trp,

P'3 : Glu, Gln, Asn, Val, Phe ou Tyr.

2. Composition de détergent selon la revendication 1, dans laquelle l'inhibiteur est un inhibiteur de la subtilisine d'orge modifié CI-1 ou CI-2, un inhibiteur de la subtilisine de pomme de terre PSI, Eglin B ou C, un inhibiteur de la subtilisine de tomate (TSI) ou un inhibiteur de la subtilisine de Vicia (VSI).

3. Composition de détergent selon l'une quelconque des revendications 1 ou 2, dans laquelle la protéase est une protéase sérine, de préférence une protéase alcaline microbienne ou une protéase analogue à la trypsine.

4. Composition de détergent selon la revendication 3, dans laquelle la protéase alcaline microbienne est une subtilisine.

5. Composition de détergent selon la revendication 4, dans laquelle la subtilisine est dérivée de Bacillus et est de préférence la subtilisine Novo, la subtilisine Carlsberg, BPN', la subtilisine 309, la subtilisine 147 ou la subtilisine 168.

6. Composition de détergent liquide aqueux selon l'une quelconque des revendications précédentes.

7. Additif de détergent comportant une protéase sous la forme d'un liquide stabilisé ou d'un granulé non-poussiéreux, caractérisé en ce qu'il comporte un inhibiteur réversible de protéase de type peptidique ou protéique dans lequel l'inhibiteur est un inhibiteur modifié de la subtilisine de la famille VI ayant une ou plusieurs des substitutions d'acides aminés suivantes au niveau de la position indiquée :

P6 : Ala, Glu ou Lys

P5 : Gly, Val, Leu ou Pro

P4 : Val, Pro, Trp, Ser, Glu ou Arg

P3 : Tyr, Glu, Ala, Arg, Pro, Ser, Lys ou Trp,

P2 : Ser, Lys, Arg, Pro, Glu, Val, Tyr, Trp ou Ala

P1 : Arg, Tyr, Pro, Trp, Glu, Val, Ser, Lys ou Ala

P'1 : Gln, Ser, Thr, Ile, Lys ou Pro,

P'2 : Val, Glu, Arg, Pro ou Trp,

P'3 : Glu, Gln, Asn, Val, Phe ou Tyr.

8. Additif de détergent selon la revendication 7, dans lequel l'inhibiteur est un inhibiteur de subtilisine CI-1 ou CI-2 d'orge modifié, un inhibiteur de la subtilisine de pomme de terre PSI, un Eglin B ou C, un inhibiteur de la subtilisine de tomate (TSI) ou un inhibiteur de la subtilisine de Vicia (VSI).

9. Procédé pour stabiliser une protéase par l'incorporation d'un inhibiteur de protéase de type peptidique ou protéique, caractérisé en ce que l'inhibiteur est un inhibiteur de la subtilisine modifié de la famille VI ayant une ou plusieurs des substitutions d'acides aminées suivantes au niveau de la position indiquée :

P6 : Ala, Glu ou Lys

P5 : Gly, Val, Leu ou Pro

P4 : Val, Pro, Trp, Ser, Glu ou Arg,

P3 : Tyr, Glu, Ala, Arg, Pro, Ser, Lys ou Trp,

P2 : Ser, Lys, Arg, Pro, Glu, Val, Tyr, Trp ou Ala

P1 : Arg, Tyr, Pro, Trp, Glu, Val, Ser, Lys ou Ala

P'1 : Gln, Ser, Thr, Ile, Lys ou Pro,

P'2 : Val, Glu, Arg, Pro ou Trp,

P'3 : Glu, Gln, Asn, Val, Phe ou Tyr.

10. Procédé selon la revendication 9, dans lequel l'inhibiteur est un inhibiteur de la subtilisine d'orge CI-1 ou CI-2 modifié, un inhibiteur la subtilisine de pomme de terre PSI, un Eglin B ou C, un inhibiteur de la subtilisine de tomate (TSI) ou un inhibiteur de la subtilisine de <u>Vicia</u> (VSI).

11. Inhibiteur de la subtilisine modifié de la famille VI ayant une ou plusieurs des substitutions d'acides aminés suivantes au niveau de la position indiquée :

> P6 : Ala, Glu ou Lys
> P5 : Gly, Val, Leu ou Pro
> P4 : Val, Pro, Trp, Ser, Glu ou Arg
> P3 : Tyr, Glu, Ala, Arg, Pro, Ser, Lys ou Trp,
> P2 : Ser, Lys, Arg, Pro, Glu, Val, Tyr, Trp ou Ala
> P1 : Arg, Tyr, Pro, Trp, Glu, Val, Ser, Lys ou Ala
> P'1 : Gln, Ser, Thr, Ile, Lys ou Pro,
> P'2 : Val, Glu, Arg, Pro ou Trp,
> P'3 : Glu, Gln, Asn, Val, Phe ou Tyr

excluant :

> Eglin B ou C substitué par
> Pro en position 42 (P4)
> Ser ou Pro en position 44 (P2),
> Leu, Arg, Phe, Tyr en position 45 (P1) ou
> Glu, Gln Ser ou Thr au niveau de 46 (P'1),
> Eglin C substitué par Arg 45, Ser 46,
> CI-2 substitué par Tyr, Ala ou Lys au niveau de 59 (P1) et
> CI-1B substitué par Pro en P2 et Asn en P'3.

12. Inhibiteur de la subtilisine d'orge CI-1 ou CI-2 modifié, inhibiteur la subtilisine de pomme de terre PSI, Eglin B ou C, inhibiteur de la subtilisine de tomate (TSI) ou inhibiteur de la subtilisine de <u>Vicia</u> (VSI) selon la revendication 11.

13. Molécule d'ADN recombinant comportant une séquence nucléotidique codant pour l'inhibiteur de la subtilisine modifié selon les revendications 11 ou 12.

14. Organisme hôte transformé comportant l'ADN selon la revendication 13.

15. Procédé de production de l'inhibiteur selon les revendications 11 ou 12, caractérisé en ce qu'il comporte la culture de l'organisme hôte transformé selon la revendication 14.

Fig. 1

Fig. 2

Fig. 3